# EUROPEAN PATENT SPECIFICATION

(11) **EP 4 253 569 B1**
(45) Date of publication and mention of the grant of the patent: **22.10.2025**
(21) Application number: 23180518.5
(22) Date of filing: 27.08.2020
(51) Int. Cl.: C12N 15/52, C12N 15/54, C12N 15/70, C12N 9/00, C12N 9/10, C12N 1/21, C12P 13/08, C12R 1/19, C12N 9/16, C07K 14/245, C12N 9/12

(54) **ESCHERICHIA COLI-BASED RECOMBINANT STRAIN, CONSTRUCTION METHOD THEREFOR AND USE THEREOF**
REKOMBINANTER STAMM AUF ESCHERICHIA-COLI-BASIS, VERFAHREN ZU SEINER HERSTELLUNG UND SEINE VERWENDUNG
SOUCHE RECOMBINANTE À BASE D'ESCHERICHIA COLI, SA MÉTHODE DE CONSTRUCTION ET SON UTILISATION

(30) Priority: 28.08.2019 CN 201910804679; 28.08.2019 CN 201910804688; 27.09.2019 CN 201910926295
(43) Date of publication of application: 04.10.2023
(62) Divisional of application: 20859580.1
(73) Proprietor: Heilongjiang Eppen Biotech Co., Ltd., Daqing, Heilongjiang 166200 (CN)
(72) Inventor: WEI, Aiying, 166200 Daqing (CN); MENG, Gang, 166200 Daqing (CN); JIA, Huiping, 166200 Daqing (CN); GAO, Xiaohang, 166200 Daqing (CN); MA, Fengyong, 166200 Daqing (CN); ZHOU, Xiaoqun, 166200 Daqing (CN); ZHAO, Chunguang, 166200 Daqing (CN); YANG, Lipeng, 166200 Daqing (CN); SU, Houbo, 166200 Daqing (CN)
(74) Representative: Huang, Liwei

(56) References cited:
- EP-A1- 2 628 792
- CN-A- 107 267 568
- CN-A- 110 564 742
- CN-A- 110 592 109
- DATABASE Geneseq [online] 27 March 2014 (2014-03-27), "E. coli (p)ppGpp synthetase II coding sequence mutant 84 insHD, SEQ ID 7.", XP002807372, retrieved from EBI accession no. GSN:BAS58321 Database accession no. BAS58321
- LEE KWANG HO ET AL: "Systems metabolic engineering of Escherichia coli for L-threonine production", MOLECULAR SYSTEMS BIOLOGY, THE MACMILLAN BUILDING, LONDON, GB, vol. 3, no. 149, 1 January 2007 (2007-01-01), pages 1 - 8, XP002603175, ISSN: 1744-4292, [retrieved on 20071204], DOI: 10.1038/MSB4100196
- MARTIN JULIA E. ET AL: "The Escherichia coli Small Protein MntS and Exporter MntP Optimize the Intracellular Concentration of Manganese", PLOS GENETICS, vol. 11, no. 3, 16 March 2015 (2015-03-16), US, pages e1004977, XP93140850, ISSN: 1553-7404, Retrieved from the Internet <URL:https://www.ncbi.nlm.nih.gov/pmc/articles/PMC4361602/pdf/pgen.1004977.pdf> DOI: 10.1371/journal.pgen.1004977
- DATABASE GenPept [online] 19 June 2019 (2019-06-19), MARTIN J. E. ET AL: "Manganese efflux pump MntP [Escherichia coli] -Protein -NCBI", XP93140842, retrieved from https://www.ncbi.nlm.nih.gov/protein/WP_001393446 Database accession no. WP_001393446
- LINK A J ET AL: "METHODS FOR GENERATING PRECISE DELETIONS AND INSERTIONS IN THE GENOME OF WILD-TYPE ESCHERICHIA COLI: APPLICATION TO OPEN READING FRAME CHARACTERIZATION", JOURNAL OF BACTERIOLOGY, AMERICAN SOCIETY FOR MICROBIOLOGY, US, vol. 179, no. 20, 1 October 1997 (1997-10-01), pages 6228 - 6237, XP000890089, ISSN: 0021-9193

## Description

The present application claims priority to Chinese Patent Application No. 2019109262958 filed with China National Intellectual Property Administration on Sep. 27, 2019 and Chinese Patent Application No. 2019108046792 on Aug. 28, 2019 as well as Chinese Patent Application No. 2019108046881 on Aug. 28, 2019.

### TECHNICAL FIELD

The present invention relates to the technical field of genetic engineering and microorganisms, and in particular to a *spoT* gene, a recombinant protein, a recombinant strain, a construction method therefor and use thereof.

### BACKGROUND

L-threonine is one of the eight essential amino acids, and is an amino acid that humans and animals cannot synthesize on their own. L-threonine can strengthen the absorption of grains, regulate the metabolism balance *in vivo* and promote the growth and development of organisms, and thus is widely applied to the feed, medicine and food industries.

At present, L-threonine can be produced mainly via a chemical synthesis method, a protein hydrolysis method and a microbial fermentation method, wherein the microbial fermentation method has the advantages of low production cost, high production intensity and small environmental pollution, thereby becoming the most widely applied method for industrially producing L-threonine. Various bacteria can be used for microbial fermentation production of L-threonine, such as mutants obtained by wild-type induction of Escherichia coli (*E. coli*)*,* Corynebacterium, and Serratia, as production strains. Specific examples include amino acid analogue resistant mutants or various auxotrophs, such as methionine, threonine, and isoleucine. However, in the conventional mutation breeding, the strain grows slowly and generates more byproducts due to random mutation, so that a high-yield strain is not easy to obtain. Therefore, the construction of recombinant *E. coli* by metabolic engineering is an effective way to produce L-threonine. At present, overexpression or attenuation of key enzyme genes in the amino acid synthesis pathway and the competitive pathway mediated by expression plasmids is a main means for genetic modification of *E. coli.* There is still a need to develop a method for producing L-threonine more economically with a high yield.

*E. coli,* as a host for exogenous gene expression, has the advantages of clear genetic background, simple technical operation and culture conditions and economic large-scale fermentation, and thus is paid more attention by genetic engineering experts. The genome DNA of *E. coli* is a circular molecule in a nucleoid, and a plurality of circular plasmid DNAs can also be provided. A nucleoid in cells of *E. coli* has one DNA molecule with a length of about 4,700,000 base pairs, and have about 4400 genes distributed on the DNA molecule, with each gene having an average length of about 1000 base pairs. For the strains of *E. coli* commonly used in molecular biology, the most commonly used strains in DNA recombination experiments, with a few exceptions, are an *E. coli* K12 strain and a derivative thereof.

Some prior arts can be seen from the following documents.
D1: EP 2628792 A1(EVONIK INDUSTRIES AG [DE]) 21 August 2013 (2013-08-21)
& DATABASE Geneseq [Online] 27 March 2014 (2014-03-27), "E. coli (p)ppGpp synthetase II coding sequence mutant 84 insHD, SEQ ID 7.", XP002807372, retrieved from EBI accession no. GSN:BAS58321, Database accession no. BAS58321
D2: CN 107267568 A (XUZHOU INST TECHNOLOGY) 20 October 2017(2017-10-20)
D3: LEE KWANG HO ET AL: "Systems metabolic engineering of Escherichia coli for L-threonine production", MOLECULAR SYSTEMS BIOLOGY, THE MACMILLAN BUILDING, LONDON, GB, vol.3, no.149, 1 January 2007 (2007-01-01), pages 1-8, XP002603175
D4: Martin Julia E. ET AL: "The Escherichia coli Small Protein MntS and Exporter MntP Optimize the Intracellular Concentration of Manganese", PLoS Genetics, vol. 11, no. 3, 16 March 2015(2015-03-16), page e1004977, XP93140850
&DATABASE GenPept [Online] 19 June 2019 (2019-06-19), Martin J. E. ET AL: "Manganese efflux pump MntP [Escherichia coli]-Protein-NCBI", XP93140842, Database accession no. WP_001393446

### SUMMARY

The present invention is set out in the appended set of claims.

Provided is an Escherichia coli strain K12-based recombinant strain or a derivative strain thereof, a recombinant construction method therefor and use thereof in the fermentation production of an amino acid.

It is focused on a wild-type kdtA gene (ORF sequence is shown in a sequence 73556-74833 in GenBank accession No. CP032667.1), a wild-type *spoT* gene (ORF sequence is shown in a sequence 3815907-3818015 in GenBank accession No. AP009048.1) and a wild-type *yebN* gene (ORF sequence is shown in a sequence 1907402-1907968 in GenBank accession No. AP009048.1) of an *E. coli* K12 strain and a derivative strain thereof (such as MG1655 and W3110), and finds that a mutant gene obtained by subjecting the gene to site-directed mutagenesis and a recombinant strain comprising the gene can be used for the production of L-threonine, and compared with an unmutated wild-type strain, the obtained strain can greatly improve the yield of L-threonine and has good strain stability, and also has lower production cost as an L-threonine production strain.

For the first technical solution, which is not covered by the claims, provided is a nucleotide sequence comprising a sequence formed by a mutation occurring at the 82^{th} base of a coding sequence of a wild-type *kdtA* gene shown in SEQ ID NO: 1.

The mutation refers to a change in a base/nucleotide at the site, and the mutation method may be at least one selected from mutagenesis, PCR site-directed mutagenesis, and/or homologous recombination, and the like.

The mutation is that guanine (G) mutates to adenine (A) at the 82^{th} base in SEQ ID NO: 1; specifically, the mutated nucleotide sequence is shown in SEQ ID NO: 2.

Provided is a recombinant protein encoded by the above-mentioned nucleotide sequence.

The recombinant protein disclosed herein comprises an amino acid sequence shown in SEQ ID NO: 4.

Provided is a recombinant vector comprising the above-mentioned nucleotide sequence or the recombinant protein.

The recombinant vector disclosed herein is constructed by introducing the above-mentioned nucleotide sequence into a plasmid; as an embodiment, the plasmid is a pKOV plasmid. Specifically, the nucleotide sequence and the plasmid may be digested with an endonuclease to form complementary cohesive ends which are ligated to construct a recombinant vector.

Provided is a recombinant strain, which comprises a *kdtA* gene coding nucleotide sequence having a point mutation occurring at the coding sequence.

The recombinant strain disclosed herein contains the above-mentioned nucleotide sequence.

The recombinant strain contains the nucleotide sequence shown in SEQ ID NO: 2.

The recombinant strain contains the amino acid sequence shown in SEQ ID NO: 4.

The recombinant strain disclosed herein is formed by introducing the above-mentioned recombinant vector into a host strain; the host strain is not particularly defined, and may be selected from a L-threonine production strain known in the art that retains the *kdtA* gene, for example, from Escherichia coli. As an embodiment, the host strain is an *E. coli* K12 (W3110) strain, or an *E. coli* CGMCC 7.232 strain.

The recombinant strain disclosed herein takes a pKOV plasmid as a vector.

The recombinant strain may or may not further comprise other modifications.

Provided is a construction method for a recombinant strain, which comprises the following step: modifying a nucleotide sequence of a wild-type *kdtA* gene coding region shown in SEQ ID NO: 1 to enable a mutation to occur at the 82^{th} base of the sequence so as to obtain a L-threonine production recombinant strain comprising the mutated *kdtA* coding gene.

According to the construction method, the modification comprises at least one of mutagenesis, PCR site-directed mutagenesis, and/or homologous recombination, and the like.

According to the construction method, the mutation is that guanine (G) mutates to adenine (A) at the 82^{th} base in SEQ ID NO: 1; specifically, the mutated nucleotide sequence is shown in SEQ ID NO: 2.

Furthermore, the construction method comprises the following steps:
(1) modifying a nucleotide sequence of an open reading frame region of the wild-type kdtA gene shown in SEQ ID NO: 1 to enable a mutation to occur at the 82^{th} base of the sequence so as to obtain a mutated nucleotide sequence of an open reading frame region of the *kdtA* gene;
(2) ligating the mutated nucleotide sequence to a plasmid to construct a recombinant vector; and
(3) introducing the recombinant vector into a host strain to obtain the L-threonine production recombinant strain having a point mutation.

According to the construction method, the step (1) comprises: the construction of the *kdtA* gene coding region having a point mutation, namely comprising synthesizing two pairs of primers for amplifying *kdtA* gene coding region fragments according to the *kdtA* gene coding sequence, and introducing the point mutation in the wild-type *kdtA* gene coding region (SEQ ID NO: 1) by PCR site-directed mutagenesis to obtain a nucleotide sequence (SEQ ID NO: 2) of the *kdtA* gene coding region having the point mutation, wherein the nucleotide sequence is marked as *kdtA*^{(G82A)}.

In an embodiment, in the step (1), the primers are:
P1: 5' CGGGATCCACCAGTGAACCGCCAACA 3' (SEQ ID NO: 5);
P2: 5' TGCGCGGACGTAAGACTC 3' (SEQ ID NO: 6);
P3: 5' GAGTCTTACGTCCGCGCA 3' (SEQ ID NO: 7); and
P4: 5' AAGGAAAAAAGCGGCCGCTTCCCGCACCTTTATTG 3' (SEQ ID NO: 8).

In an embodiment, the step (1) comprises: using primers P1/P2 and P3/P4 for PCR amplification by taking *E. coli* K12 as a template to obtain two isolated DNA fragments (*kdtA* Up and *kdtA* Down) having a length of 927 bp and 695 bp and *kdtA* gene coding regions; and separating and purifying the two DNA fragments by agarose gel electrophoresis, and then performing overlap PCR by taking P1 and P4 as primers and taking the two DNA fragments as templates to obtain *kdtA*^{G82A}-Up-Down.

In an embodiment, the nucleotide sequence of the *kdtA*^{G82A}-Up-Down has a length of 1622 bp.

In an embodiment, the PCR amplification is performed as follows: denaturing at 94 °C for 30 s, annealing at 52 °C for 30 s, and extending at 72 °C for 30 s (for 30 cycles).

In an embodiment, the overlap PCR amplification is performed as follows: denaturing at 94 °C for 30 s, annealing at 52 °C for 30 s, and extending at 72 °C for 60 s (for 30 cycles).

According to the construction method, the step (2) comprises: the construction of the recombinant vector, namely comprising separating and purifying the *kdtA*^{(G82A)}-Up-Down fragment by agarose gel electrophoresis, then digesting the purified fragment and the pKOV plasmid with *BamH* I/*Not* I, and separating and purifying the digested *kdtA*^{(G82A)}-Up-Down fragment and the digested pKOV plasmid by agarose gel electrophoresis followed by ligation to obtain the recombinant vector pKOV-*kdtA*^{(G82A)}.

According to the construction method, the step (3) comprises: the construction of the recombinant strain, namely comprising transforming the recombinant vector pKOV-*kdtA*^{(G82A)} into the host strain to obtain the recombinant strain.

In an embodiment, the transformation in the step (3) is an electrotransformation process; illustratively, in the step (3), the recombinant vector is introduced into the host strain.

According to the construction method, the method further comprises a step of screening the recombinant strain; illustratively, screening is performed by using a chloramphenicol culture medium.

Provided is a recombinant strain obtained by the above-mentioned construction method.

Provided is use of the recombinant strain in the preparation of L-threonine or the improvement of L-threonine fermentation volume.

The use of the recombinant strain in the preparation of L-threonine comprises fermenting the recombinant strain to prepare L-threonine.

For the second technical solution, which is not covered by the claims, provided is a nucleotide sequence comprising a nucleotide sequence formed by a mutation occurring at the 520^{th} base of the *spoT* gene coding sequence shown in SEQ ID NO: 13.

The mutation refers to a change in a base/nucleotide at the site, and the mutation method may be at least one selected from mutagenesis, PCR site-directed mutagenesis, and/or homologous recombination, and the like.

The mutation is that guanine (G) mutates to thymine (T) at the 520^{th} base in SEQ ID NO: 13.

Provided is a recombinant protein encoded by the above-mentioned nucleotide sequence.

Provided is a recombinant vector comprising the above-mentioned nucleotide sequence or the recombinant protein.

The recombinant vector disclosed herein is constructed by introducing the above-mentioned nucleotide sequence into a plasmid; as an embodiment, the plasmid is a pKOV plasmid. Specifically, the nucleotide sequence and the plasmid may be digested with an endonuclease to form complementary cohesive ends which are ligated to construct a recombinant vector.

Provided is a recombinant strain, which comprises a *spoT* gene coding nucleotide sequence having a point mutation on the coding sequence, for example, a *spoT* gene coding nucleotide sequence shown in SEQ ID NO: 13 having a point mutation occurring at the 520^{th} base.

The mutation is that guanine (G) mutates to thymine (T) at the 520^{th} base in SEQ ID NO: 13.

In the present invention, the recombinant strain contains the nucleotide sequence shown in SEQ ID NO: 14.

In the present invention, the recombinant strain contains the amino acid sequence shown in SEQ ID NO: 16.

The recombinant strain disclosed herein is formed by introducing the above-mentioned recombinant vector into a host strain; the host strain is not particularly defined, and may be selected from a L-threonine production strain known in the art that retains the *spoT* gene, for example, from Escherichia coli. As an embodiment, the host strain is an *E. coli* K12 (W3110) strain, or an *E. coli* CGMCC 7.232 strain.

The recombinant strain disclosed herein takes a pKOV plasmid as a vector.

The recombinant strain may or may not further comprise other modifications.

Provided is a construction method for a recombinant strain, which comprises the following step: modifying a nucleotide sequence of a *spoT* gene coding region shown in SEQ ID NO: 13 to enable a mutation to occur at the 520^{th} base of the sequence so as to obtain a recombinant strain comprising the mutated *spoT* coding gene.

According to the construction method, the modification comprises at least one of mutagenesis, PCR site-directed mutagenesis, and/or homologous recombination, and the like.

According to the construction method of the present invention, the mutated nucleotide sequence is shown in SEQ ID NO: 14.

Furthermore, the construction method comprises the following steps:
(1) modifying a nucleotide sequence of an open reading frame region of the wild-type spoT gene shown in SEQ ID NO: 13 to enable a mutation to occur at the 520^{th} base of the sequence so as to obtain a mutated nucleotide sequence;
(2) ligating the mutated nucleotide sequence to a plasmid to construct a recombinant vector; and
(3) introducing the recombinant vector into a host strain to obtain the recombinant strain having a point mutation.

According to the construction method of the present invention, the step (1) comprises: the construction of the *spoT* gene coding region having a point mutation, namely comprising synthesizing two pairs of primers for amplifying *spoT* gene coding region fragments according to the *spoT* gene coding sequence, and introducing the point mutation in the wild-type *spoT* gene coding region (SEQ ID NO: 13) by PCR site-directed mutagenesis to obtain a nucleotide sequence (SEQ ID NO: 14) of the *spoT* gene coding region having the point mutation, wherein the nucleotide sequence is marked as *spoT^{(G520T)}.*

In an embodiment, in the step (1), the primers are:
P1: 5' CGGGATCCGAACAGCAAGAGCAGGAAGC 3' (SEQ ID NO: 17);
P2: 5' TGTGGTGGATACATAAACG 3'(SEQ ID NO: 18);
P3: 5' GCACCGTTTATGTATCCACC 3'(SEQ ID NO: 19); and
P4: 5' AAGGAAAAAAGCGGCCGCACGACAAAGTTCAGCCAAGC 3' (SEQ ID NO: 20).

In an embodiment, the step (1) comprises: using primers P1/P2 and P3/P4 for PCR amplification by taking *E. coli* K12 as a template to obtain two isolated DNA fragments (*spoT^{(G520T)}*-Up and *spoT^{(G520T)}*-Down) having a length of 620 bp and 880 bp and *spoT* gene coding regions having a point mutation; and separating and purifying the two DNA fragments by agarose gel electrophoresis, and then performing overlap PCR by taking P1 and P4 as primers and taking the two DNA fragments as templates to obtain a *spoT^{G520T}*-Up-Down fragment.

In an embodiment, the nucleotide sequence of the *spoT^{(G520T)}*-Up-Down fragment has a length of 1500 bp.

In an embodiment, the PCR amplification is performed as follows: denaturing at 94 °C for 30 s, annealing at 52 °C for 30 s, and extending at 72 °C for 30 s (for 30 cycles).

In an embodiment, the overlap PCR amplification is performed as follows: denaturing at 94 °C for 30 s, annealing at 52 °C for 30 s, and extending at 72 °C for 60 s (for 30 cycles).

According to the construction method, the step (2) comprises: the construction of the recombinant vector, namely comprising separating and purifying the *spoT^{(G520T)}*-Up-Down fragment by agarose gel electrophoresis, then digesting the purified fragment and the pKOV plasmid with *BamH* I/*Not* I, and separating and purifying the digested *spoT^{(G520T)}*-Up-Down fragment and the digested pKOV plasmid by agarose gel electrophoresis followed by ligation to obtain the recombinant vector pKOV-*spoT^{(G520T)}.*

According to the construction method, the step (3) comprises: the construction of the recombinant strain, namely comprising introducing the recombinant vector *pKOV-spoT^{(G520T)}* into the host strain to obtain the recombinant strain.

In an embodiment, the introduction in the step (3) is an electrotransformation process.

According to the construction method, the method further comprises a step of screening the recombinant strain; illustratively, screening is performed by using a chloramphenicol culture medium.

Provided is a recombinant strain obtained by the above-mentioned construction method.

Provided is use of the above-mentioned recombinant strain in the preparation of L-threonine.

The use of the nucleotide sequence, the recombinant protein, the recombinant vector and the recombinant strain in the preparation of L-threonine comprises fermenting the recombinant strain to prepare L-threonine.

For the third technical solution, which is not covered by the claims, provided is a nucleotide sequence comprising a nucleotide sequence formed by a mutation occurring at the 74^{th} base of a wild-type *yebN* gene coding sequence shown in SEQ ID NO: 23.

The mutation is that guanine (G) mutates to adenine (A) at the 74^{th} base in SEQ ID NO: 23; specifically, the nucleotide sequence is shown in SEQ ID NO: 24. The mutation refers to a change in a base/nucleotide at the site, and the mutation method may be at least one selected from mutagenesis, PCR site-directed mutagenesis, and/or homologous recombination, and the like.

Provided is a recombinant protein encoded by the above-mentioned nucleotide sequence.

The recombinant protein disclosed herein comprises an amino acid sequence shown in SEQ ID NO: 26; specifically, the recombinant protein comprises a substitution of glycine with aspartic acid at the 25^{th} site of an amino acid sequence shown in SEQ ID NO: 25.

Provided is a recombinant vector comprising the above-mentioned nucleotide sequence or the recombinant protein.

The recombinant vector disclosed herein is constructed by introducing the above-mentioned nucleotide sequence into a plasmid; as an embodiment, the plasmid is a pKOV plasmid. Specifically, the nucleotide sequence and the plasmid may be digested with an endonuclease to form complementary cohesive ends which are ligated to construct a recombinant vector.

Provided is a recombinant strain, which comprises a *yebN* gene coding nucleotide sequence having a point mutation on the coding sequence, for example, a *yebN* gene coding nucleotide sequence shown in SEQ ID NO: 23 having a point mutation occurring at the 74^{th} base.

According to the recombinant strain, the *yebN* gene coding nucleotide sequence comprises a mutation that guanine (G) mutates to adenine (A) at the 74^{th} base in SEQ ID NO: 23.

As an embodiment, the recombinant strain contains the nucleotide sequence shown in SEQ ID NO: 24.

As an embodiment, the recombinant strain contains the amino acid sequence shown in SEQ ID NO: 26.

The recombinant strain disclosed herein is formed by introducing the above-mentioned recombinant vector into a host strain; the host strain is not particularly defined, and may be selected from a L-threonine production strain known in the art that retains the *yebN* gene, for example, from Escherichia coli. As an embodiment, the host strain is *E. coli* K12, a derivative strain thereof *E. coli* K12 (W3110), or an *E. coli* CGMCC 7.232 strain.

The recombinant strain disclosed herein takes a pKOV plasmid as a vector.

The recombinant strain may or may not further comprise other modifications.

Provided is a construction method for a recombinant strain, which comprises the following step: modifying a nucleotide sequence of a wild-type *yebN* gene coding region shown in SEQ ID NO: 23 to enable a mutation to occur at the 74^{th} base of the sequence so as to obtain a recombinant strain comprising the mutated *yebN* coding gene.

According to the construction method, the modification comprises at least one of mutagenesis, PCR site-directed mutagenesis, and/or homologous recombination, and the like.

According to the construction method, the mutation is that guanine (G) mutates to adenine (A) at the 74^{th} base in SEQ ID NO: 23; specifically, the mutated nucleotide sequence is shown in SEQ ID NO: 24.

Furthermore, the construction method comprises the following steps:
(1) modifying a nucleotide sequence of an open reading frame region of the wild-type *yebN* gene shown in SEQ ID NO: 23 to enable a mutation to occur at the 74^{th} base of the sequence so as to obtain a mutated nucleotide sequence of the open reading frame region of the *yebN* gene;
(2) ligating the mutated nucleotide sequence to a plasmid to construct a recombinant vector; and
(3) introducing the recombinant vector into a host strain to obtain the recombinant strain having a point mutation.

According to the construction method, the step (1) comprises: the construction of the *yebN* gene coding region having a point mutation, namely comprising synthesizing two pairs of primers for amplifying *yebN* gene coding region fragments according to the *yebN* gene coding sequence, and introducing the point mutation in the wild-type *yebN* gene coding region (SEQ ID NO: 23) by PCR site-directed mutagenesis to obtain a nucleotide sequence (SEQ ID NO: 24) of the *yebN* gene coding region having the point mutation, wherein the nucleotide sequence is marked as *yebN^{(G74A)}.*

In an embodiment, in the step (1), the primers are:
P1: 5' CGGGATCCCTTCGCCAATGTCTGGATTG 3' (SEQ ID NO: 27);
P2: 5' ATGGAGGGTGGCATCTTTAC 3'(SEQ ID NO: 28);
P3: 5' TGCATCAATCGGTAAAGATG 3' (SEQ ID NO: 29); and
P4: 5' AAGGAAAAAAGCGGCCGCCAACTCCGCACTCTGCTGTA 3' (SEQ ID NO: 30).

In an embodiment, the step (1) comprises: using primers P1/P2 and P3/P4 for PCR amplification by taking *E. coli* K12 as a template to obtain two isolated DNA fragments (*yebN^{(G74A)}*-Up and *yebN^{(G74A)}-*Down) having a length of 690 bp and 700 bp and *yebN* gene coding regions having a point mutation; and separating and purifying the two DNA fragments by agarose gel electrophoresis, and then performing overlap PCR by taking P1 and P4 as primers and taking the two DNA fragments as templates to obtain a *yebN^{G74A}*-Up-Down fragment.

In an embodiment, the nucleotide sequence of the *yebN^{(G74A)}*-Up-Down fragment has a length of 1340 bp.

In an embodiment, the PCR amplification is performed as follows: denaturing at 94 °C for 30 s, annealing at 52 °C for 30 s, and extending at 72 °C for 30 s (for 30 cycles).

In an embodiment, the overlap PCR amplification is performed as follows: denaturing at 94 °C for 30 s, annealing at 52 °C for 30 s, and extending at 72 °C for 60 s (for 30 cycles).

According to the construction method, the step (2) comprises: the construction of the recombinant vector, namely comprising separating and purifying the *yebN^{(G74A)}*-Up-Down fragment by agarose gel electrophoresis, then digesting the purified fragment and the pKOV plasmid with *Bam*H I/*Not* I, and separating and purifying the digested *yebN^{(G74A)}*-Up-Down fragment and the digested pKOV plasmid by agarose gel electrophoresis followed by ligation to obtain the recombinant vector pKOV*-yebN^{(G74A)}.*

According to the construction method, the step (3) comprises: the construction of the recombinant strain, namely comprising introducing the recombinant vector pKOV-*yebN^{(G74A)}* into the host strain to obtain the recombinant strain.

In an embodiment, the introduction in the step (3) is an electrotransformation process.

According to the construction method, the method further comprises a step of screening the recombinant strain; illustratively, screening is performed by using a chloramphenicol culture medium.

Provided is a recombinant strain obtained by the above-mentioned construction method.

Provided is use of the above-mentioned nucleotide sequence, the recombinant protein, the recombinant vector and the recombinant strain in the preparation of L-threonine.

The use of the recombinant strain in the preparation of L-threonine comprises fermenting the recombinant strain to prepare L-threonine.

### DETAILED DESCRIPTION

The above-mentioned and other features and advantages of the present invention are explained and illustrated in more detail in the following description of examples.

Unless otherwise indicated, the materials and reagents herein are either commercially available or can be prepared by one skilled in the art in light of the prior art.

### Example 1 (not being covered by the claims)

### (1) Construction of Plasmid pKOV-kdtA^{(G82A)} with kdtA Gene Coding Region Having Site-Directed Mutation (G82A)

(equivalent to that alanine is substituted with threonine at the 28^{th} site (A28T) in a wild-type protein-coding amino acid sequence SEQ ID NO: 3, the substituted amino acid sequence being SEQ ID NO: 4)

The 3-deoxy-D-mannose-sulfamyltransferase was encoded by a *kdtA* gene, and in an *E. coli* K12 strain and a derivative strain thereof (such as MG1655), an ORF sequence of the wild-type *kdtA* gene is shown in a sequence 73556-74833 in Genbank accession No. CP032667.1. Two pairs of primers for amplifying *kdtA* were designed and synthesized according to the sequence, and a vector was constructed for a base G mutating to a base A at the 82^{th} site in a *kdtA* gene coding region sequence of an original strain. The primers (synthesized by Shanghai Invitrogen Corporation) were designed as follows:
P1: 5' CGGGATCCACCAGTGAACCGCCAACA 3' (SEQ ID NO: 5);
P2: 5' TGCGCGGACGTAAGACTC 3' (SEQ ID NO: 6);
P3: 5' GAGTCTTACGTCCGCGCA 3' (SEQ ID NO: 7); and
P4: 5' AAGGAAAAAAGCGGCCGCTTCCCGCACCTTTATTG 3' (SEQ ID NO: 8).

The construction method was as follows: using primers P1/P2 and P3/P4 for PCR amplification by taking a genome of a wild-type strain *E. coli* K12 as a template to obtain two DNA fragments having a length of 927 bp and 695 bp and point mutation (*kdtA^{(G82A})*-Up and *kdtA^{(G82A)}*-Down fragments). The PCR amplification was performed as follows: denaturing at 94 °C for 30 s, annealing at 52 °C for 30 s, and extending at 72 °C for 30 s (for 30 cycles). The two DNA fragments were separated and purified by agarose gel electrophoresis, and then the two purified DNA fragments were taken as templates, and P1 and P4 were taken as primers to perform overlap PCR to obtain a fragment (*kdtA*^{(G82A)}-Up-Down) having a length of about 1622 bp. The overlap PCR amplification was performed as follows: denaturing at 94 °C for 30 s, annealing at 52 °C for 30 s, and extending at 72 °C for 60 s (for 30 cycles). The *kdtA*^{(G82A)}-Up-Down fragment was separated and purified by agarose gel electrophoresis, then the purified fragment and a pKOV plasmid (purchased from Addgene) were digested with *Bam*H I/*Not* I, and the digested *kdtA*^{(G82A)}-Up-Down fragment and the digested pKOV plasmid were separated and purified by agarose gel electrophoresis followed by ligation to obtain a vector pKOV-*kdtA*^{(G82A)}. The vector pKOV-*kdtA*^{(G82A)} was sent to a sequencing company for sequencing and identification, the result is shown in SEQ ID NO: 11, and the vector pKOV-*kdtA*^{(G82A)} with the correct point mutation (*kdta*^{(G82A)}) was stored for later use.

### (2) Construction of Engineered Strain with kdtA^{(G82A)} Having Point Mutation

A wild-type *kdtA* gene was reserved on chromosomes of a wild-type Escherichia coli strain *E*. *coli* K12 (W3110) and a high-yield L-threonine production strain *E. coli* CGMCC 7.232 (preserved in China General Microbiological Culture Collection Center). The constructed plasmid pKOV-*kdtA*^{(G82A)} was transferred into *E. coli* K12 (W3110) and *E. coli* CGMCC 7.232, respectively, and through allele replacement, the base G mutated to the base A at the 82^{th} site of the *kdtA* gene sequences in the chromosomes of the two strains. The specific process was as follows: transforming the plasmid pKOV*-kdtA(G82A)* into host bacterium competent cells through an electrotransformation process, and adding the cells into 0.5 mL of a SOC liquid culture medium; resuscitating the mixture in a shaker at 30 °C and 100 rpm for 2 h; coating an LB solid culture medium having a chloramphenicol content of 34 mg/mL with 100 µL of the culture solution, and culturing at 30 °C for 18 h; selecting grown monoclonal colonies, inoculating the colonies in 10 mL of an LB liquid culture medium, and culturing at 37 °C and at 200 rpm for 8 h; coating an LB solid culture medium having a chloramphenicol content of 34 mg/mL with 100 µL of the culture solution, and culturing at 42 °C for 12 h; selecting 1-5 single colonies, inoculating the colonies in 1 mL of an LB liquid medium, and culturing at 37 °C and 200 rpm for 4 h; coating an LB solid culture medium containing 10% of sucrose with 100 uL of the culture solution, and culturing at 30 °C for 24 h; selecting monoclonal colonies, and streaking the colonies on an LB solid culture medium having a chloramphenicol content of 34 mg/mL and an LB solid culture medium in a one-to-one correspondence manner; and selecting strains which grew on the LB solid culture medium and could not grow on the LB solid culture medium having the chloramphenicol content of 34 mg/mL for PCR amplification identification. The primers (synthesized by Shanghai Invitrogen Corporation) for use in PCR amplification were as follows:
P5: 5' CTTCCCGAAAGCCGATTG 3' (SEQ ID NO: 9); and
P6: 5' ACAAAATATACTTTAATC 3' (SEQ ID NO:10).

SSCP (Single-Strand Conformation Polymorphism) electrophoresis was performed on the PCR-amplified product; the amplified fragment of the plasmid pKOV-*kdtA*^{(G82A)} was taken as a positive control, the amplified fragment of the wild-type Escherichia coli was taken as a negative control, and water was taken as a blank control. In SSCP electrophoresis, single-stranded oligonucleotide chains having the same length but different sequence arrangements formed different spatial structures in an ice bath and also had different mobilities during electrophoresis. Therefore, the fragment electrophoresis position was not consistent with that of negative control, and a strain having a fragment electrophoresis position consistent with that of positive control is the successfully allele-replaced strain. PCR amplification was performed on the target fragment by taking the successfully allele-replaced strain as a template and using primers P5 and P6, and then the target fragment was ligated to a pMD19-T vector for sequencing. Through sequence comparison of a sequencing result, a recon formed by the base G mutating to the base A at the 82^{th} site in the *kdtA* gene coding region sequence is the successfully modified strain, and the sequencing result is shown in SEQ ID NO: 12. The recon derived from *E. coli* K12 (W3110) was named as YPThr07, and the recon derived from *E. coli* CGMCC 7.232 was named as YPThr 08.

### (3) Threonine Fermentation Experiment

The *E. coli* K12 (W3110) strain, the *E. coli* CGMCC 7.232 strain, and the mutant strains YPThr07 and YPThr08 were inoculated in 25 mL of a liquid culture medium described in Table 1, respectively, and cultured at 37 °C and 200 rpm for 12 h. Then, 1 mL of the resulting culture of each strain was inoculated in 25 mL of a liquid culture medium described in Table 1, and subjected to fermentation culture at 37 °C and 200 rpm for 36 h. The content of L-threonine was determined by HPLC, three replicates of each strain were taken, the average was calculated, and the results are shown in Table 2.

**Table 1 Culture medium formula**

| Component | Formula g/L |
|---|---|
| Glucose | 40 |
| Ammonium sulfate | 12 |
| Potassium dihydrogen phosphate | 0.8 |
| Magnesium sulfate heptahydrate | 0.8 |
| Ferrous sulfate heptahydrate | 0.01 |
| Manganese sulfate monohydrate | 0.01 |
| Yeast extract | 1.5 |
| Calcium carbonate | 0.5 |
| L-methionine | 0.5 |
| pH value adjusted with potassium hydroxide | pH 7.0 |

**Table 2 Threonine fermentation results**

| Strains | Fermentation volume (g/L) | Mean value (g/L) | Multiple of improvement |
|---|---|---|---|
| *E. coli* K12 (W3110) | 0.03 | 0.03 | - |
| | 0.03 | | |
| | 0.02 | | |
| YPThr07 | 2.3 | 2.4 | 83.3 |
| | 2.5 | | |
| | 2.5 | | |
| *E. coli* CGMCC 7.232 | 15.8 | 16.1 | - |
| | 16.2 | | |
| | 16.2 | | |
| YPThr08 | 18.1 | 18.1 | 12.4% |
| | 17.9 | | |
| | 18.4 | | |

As can be seen from the results of Table 2, the substitution of alanine at the 28^{th} site of the amino acid sequence of the *kdtA* gene with threonine contributes to the improvement of the yield of L-threonine for the original strain producing L-threonine with either high or low yield.

### Example 2

### (1) Construction of Plasmid pKOV-spoT^{(G520T)} with spoT Gene Coding Region Having Site-Directed Mutation (G520T) (equivalent to that glycine is substituted with cysteine at the 174^{th} site (G174C) in a protein-coding amino acid sequence SEQ ID NO: 15, the substituted amino acid sequence being SEQ ID NO: 16)

SPOT enzyme was encoded by a *spoT* gene, and in an *E. coli* K12 strain and a derivative strain thereof (such as W3110), an ORF sequence of the wild-type *spoT* gene is shown in a sequence 3815907-3818015 in GenBank accession No. AP009048.1. Two pairs of primers for amplifying *spoT* were designed and synthesized according to the sequence, and a vector was constructed for a base G mutating to a base T at the 520^{th} site in a *spoT* gene coding region sequence of an original strain. The primers (synthesized by Shanghai Invitrogen Corporation) were designed as follows:
P1: 5' CGGGATCCGAACAGCAAGAGCAGGAAGC 3' (the underlined part is a restriction endonuclease cutting site *Bam*H I) (SEQ ID NO: 17);
P2: 5' TGTGGTGGATACATAAACG 3' (SEQ ID NO: 18);
P3: 5' GCACCGTTTATGTATCCACC 3' (SEQ ID NO: 19); and
P4: 5' AAGGAAAAAAGCGGCCGCACGACAAAGTTCAGCCAAGC 3' (the underlined part is a restriction endonuclease cutting site *Not* I) (SEQ ID NO: 20).

The construction method was as follows: using primers P1/P2 and P3/P4 for PCR amplification by taking a genome of a wild-type strain *E. coli* K12 as a template to obtain two DNA fragments having a length of 620 bp and 880 bp and point mutation (*spoT*^{(G520T)}-Up and *spoT*^{(G520T)}-Down fragments). PCR system: 10 × Ex Taq buffer 5 µL, dNTP mixture (2.5 mM each) 4 µL, Mg²⁺ (25 mM) 4 µL, primers (10 pM) 2 µL each, Ex Taq (5 U/µL) 0.25 µL, total volume 50 µL, wherein the PCR was performed as follows: denaturing at 94 °C for 30 s, annealing at 52 °C for 30 s, and extending at 72 °C for 30 s (for 30 cycles). The two DNA fragments were separated and purified by agarose gel electrophoresis, and then the two purified DNA fragments were taken as templates, and P1 and P4 were taken as primers to perform overlap PCR to obtain a fragment (*spoT*^{(G520T)}-Up-Down) having a length of about 1500 bp. PCR system: 10 × Ex Taq buffer 5 µL, dNTP mixture (2.5 mM each) 4 µL, Mg²⁺ (25 mM) 4 µL, primers (10 pM) 2 µL each, Ex Taq (5 U/µL) 0.25 µL, total volume 50 µL, wherein the overlap PCR was performed as follows: denaturing at 94 °C for 30 s, annealing at 52 °C for 30 s, and extending at 72 °C for 60 s (for 30 cycles). The *spoT*^{(G520T)}-Up-Down fragment was separated and purified by agarose gel electrophoresis, then the purified fragment and a pKOV plasmid (purchased from Addgene) were digested with *Bam*H I/*Not* I, and the digested *spoT*^{(G520T)}-Up-Down fragment and the digested pKOV plasmid were separated and purified by agarose gel electrophoresis followed by ligation with a DNA ligase to obtain a vector pKOV-spoT^{(G520T)}. The vector pKOV-*spoT*^{(G520T)} was sent to a sequencing company for sequencing and identification, and the vector pKOV-*spoT*^{(G520T)} with the correct point mutation (*spoT*^{(G520T)}) was stored for later use.

### (2) Construction of Engineered Strain with spoT^{(G520T)} Having Point Mutation

A wild-type *spoT* gene was reserved on chromosomes of a wild-type Escherichia coli strain *E*. *coli* K12 (W3110) and a high-yield L-threonine production strain *E. coli* CGMCC 7.232 (preserved in China General Microbiological Culture Collection Center). The constructed plasmid pKOV-*spoT*^{(G520T)} was transferred into *E. coli* K12 (W3110) and *E. coli* CGMCC 7.232, respectively, and through allele replacement, the base G mutated to the base T at the 520^{th} site of the *spoT* gene sequences in the chromosomes of the two strains. The specific process was as follows: transforming the plasmid pKOV-*spoT*^{(G520T)} into host bacterium competent cells through an electrotransformation process, and adding the cells into 0.5 mL of a SOC liquid culture medium; resuscitating the mixture in a shaker at 30 °C and 100 rpm for 2 h; coating an LB solid culture medium having a chloramphenicol content of 34 µg/mL with 100 µL of the culture solution, and culturing at 30 °C for 18 h; selecting grown monoclonal colonies, inoculating the colonies in 10 mL of an LB liquid culture medium, and culturing at 37 °C and at 200 rpm for 8 h; coating an LB solid culture medium having a chloramphenicol content of 34 µg/mL with 100 µL of the culture solution, and culturing at 42 °C for 12 h; selecting 1-5 single colonies, inoculating the colonies in 1 mL of an LB liquid medium, and culturing at 37 °C and 200 rpm for 4 h; coating an LB solid culture medium containing 10% of sucrose with 100 uL of the culture solution, and culturing at 30 °C for 24 h; selecting monoclonal colonies, and streaking the colonies on an LB solid culture medium having a chloramphenicol content of 34 µg/mL and an LB solid culture medium in a one-to-one correspondence manner; and selecting strains which grew on the LB solid culture medium and could not grow on the LB solid culture medium having the chloramphenicol content of 34 µg/mL for PCR amplification identification. The primers (synthesized by Shanghai Invitrogen Corporation) for use in PCR amplification were as follows:
P5: 5' ctttcgcaagatgattatgg 3' (SEQ ID NO: 21); and
P6: 5' cacggtattcccgcttcctg 3' (SEQ ID NO: 22).

PCR system: 10 × Ex Taq buffer 5 µL, dNTP mixture (2.5 mM each) 4 µL, Mg²⁺ (25 mM) 4 µL, primers (10 pM) 2 µL each, Ex Taq (5 U/µL) 0.25 µL, total volume 50 µL, wherein the PCR amplification was performed as follows: pre-denaturation at 94 °C for 5 min, (denaturing at 94 °C for 30 s, annealing at 52 °C for 30 s, and extending at 72 °C for 90 s, for 30 cycles), and over-extending at 72 °C for 10 min. SSCP (Single-Strand Conformation Polymorphism) electrophoresis was performed on the PCR-amplified product; the amplified fragment of the plasmid pKOV-*spoT*^{(G520T)} was taken as a positive control, the amplified fragment of the wild-type Escherichia coli was taken as a negative control, and water was taken as a blank control. In SSCP electrophoresis, single-stranded oligonucleotide chains having the same length but different sequence arrangements formed different spatial structures in an ice bath and also had different mobilities during electrophoresis. Therefore, the fragment electrophoresis position was not consistent with that of negative control, and a strain having a fragment electrophoresis position consistent with that of positive control is the successfully allele-replaced strain. PCR amplification was performed on the target fragment by taking the successfully allele-replaced strain as a template and using primers P5 and P6, and then the target fragment was ligated to a pMD19-T vector for sequencing. Through sequence comparison of a sequencing result, a recon formed by the base G mutating to the base T at the 520^{th} site in the *spoT* gene coding region sequence is the successfully modified strain. The recon derived from *E. coli* K12 (W3110) was named as YPThr03, and the recon derived from *E. coli* CGMCC 7.232 was named as YPThr 04.

### (3) Threonine Fermentation Experiment

The *E. coli* K12 (W3110) strain, the *E. coli* CGMCC 7.232 strain, and the mutant strains YPThr03 and YPThr04 were inoculated in 25 mL of a liquid culture medium described in Table 1, respectively, and cultured at 37 °C and 200 rpm for 12 h. Then, 1 mL of the resulting culture of each strain was inoculated in 25 mL of a liquid culture medium described in Table 1, and subjected to fermentation culture at 37 °C and 200 rpm for 36 h. The content of L-threonine was determined by HPLC, three replicates of each strain were taken, the average was calculated, and the results are shown in Table 2.

**Table 1 Culture medium formula**

| Component | Formula g/L |
|---|---|
| Glucose | 40 |
| Ammonium sulfate | 12 |
| Potassium dihydrogen phosphate | 0.8 |
| Magnesium sulfate heptahydrate | 0.8 |
| Ferrous sulfate heptahydrate | 0.01 |
| Manganese sulfate monohydrate | 0.01 |
| Yeast extract | 1.5 |
| Calcium carbonate | 0.5 |
| L-methionine | 0.5 |
| pH value adjusted with potassium hydroxide | pH 7.0 |

**Table 2 Threonine fermentation results**

| Strains | Fermentation volume (g/L) | Mean value (g/L) | Multiple of improvement |
|---|---|---|---|
| *E. coli* K12 W3110 | 0.01 | 0.02 | - |
| | 0.02 | | |
| | 0.02 | | |
| YPThr03 | 2.2 | 2.3 | 115 |
| | 2.4 | | |
| | 2.3 | | |
| *E. coli* CGMCC 7.232 | 16.0 | 16.2 | - |
| | 16.3 | | |
| | 16.2 | | |
| YPThr04 | 17.9 | 18.1 | 11.7% |
| | 18.2 | | |
| | 18.1 | | |

As can be seen from the results of Table 2, the substitution of glycine at the 174^{th} site of the amino acid sequence of the *spoT* gene with cysteine contributes to the improvement of the yield of L-threonine for the original strain producing L-threonine with either high or low yield.

### Example 3: (not being covered by the claims)

### (1) Construction of Plasmid pKOV-yebN^{(G74A)} with yebN Gene Coding Region Having Site-Directed Mutation (G74A) (equivalent to that glycine is substituted with aspartic acid at the 25^{th} site (G25D) in a protein-coding amino acid sequence SEQ ID NO: 25, the substituted amino acid sequence being SEQ ID NO: 26)

YEBN enzyme was encoded by a *yebN* gene, and in an *E. coli* K12 strain and a derivative strain thereof (such as W3110), an ORF sequence of the wild-type *yebN* gene is shown in a sequence 1907402-1907968 in GenBank accession No. AP009048.1. Two pairs of primers for amplifying *yebN* were designed and synthesized according to the sequence, and a vector was constructed for a base G mutating to a base A at the 74^{th} site in a *yebN* gene coding region sequence of an original strain. The primers (synthesized by Shanghai Invitrogen Corporation) were designed as follows:
P1: 5' CGGGATCCCTTCGCCAATGTCTGGATTG 3' (the underlined part is a restriction endonuclease cutting site *Bam*H I) (SEQ ID NO: 27);
P2: 5' ATGGAGGGTGGCATCTTTAC 3' (SEQ ID NO:28);
P3: 5' TGCATCAATCGGTAAAGATG 3' (SEQ ID NO:29); and
P4: 5' AAGGAAAAAAGCGGCCGCCAACTCCGCACTCTGCTGTA 3' (the underlined part is a restriction endonuclease cutting site *Not* I) (SEQ ID NO: 30).

The construction method was as follows: using primers P1/P2 and P3/P4 for PCR amplification by taking a genome of a wild-type strain *E. coli* K12 as a template to obtain two DNA fragments having a length of 690 bp and 700 bp and point mutation (*yebN*^{(G74A)}-Up and *yebN*^{(G74A)}-Down fragments). PCR system: 10 × Ex Taq buffer 5 µL, dNTP mixture (2.5 mM each) 4 µL, Mg²⁺ (25 mM) 4 µL, primers (10 pM) 2 µL each, Ex Taq (5 U/µL) 0.25 µL, total volume 50 µL, wherein the PCR was performed as follows: denaturing at 94 °C for 30 s, annealing at 52 °C for 30 s, and extending at 72 °C for 30 s (for 30 cycles). The two DNA fragments were separated and purified by agarose gel electrophoresis, and then the two purified DNA fragments were taken as templates, and P1 and P4 were taken as primers to perform overlap PCR to obtain a fragment (*yebN*^{(G74A)}-Up-Down) having a length of about 1340 bp.

PCR system: 10 × Ex Taq buffer 5 µL, dNTP mixture (2.5 mM each) 4 µL, Mg²⁺ (25 mM) 4 µL, primers (10 pM) 2 µL each, Ex Taq (5 U/µL) 0.25 µL, total volume 50 µL, wherein the overlap PCR was performed as follows: denaturing at 94 °C for 30 s, annealing at 52 °C for 30 s, and extending at 72 °C for 60 s (for 30 cycles).

The *yebN*^{(G74A)}-Up-Down fragment was separated and purified by agarose gel electrophoresis, then the purified fragment and a pKOV plasmid (purchased from Addgene) were digested with *BamH* I/*Not* I, and the digested *yebN*^{(G74A)}-Up-Down fragment and the digested pKOV plasmid were separated and purified by agarose gel electrophoresis followed by ligation to obtain a vector pKOV-yebN^{(G74A)}. The vector pKOV*-yebN*^{(G74A)} was sent to a sequencing company for sequencing and identification, and the vector pKOV*-yebN*^{(G74A)} with the correct point mutation (*yebN*^{(G74A)}) was stored for later use.

### (2) Construction of Engineered Strain with yebN^{(G74A)} Having Point Mutation

A wild-type *yebN* gene was reserved on chromosomes of a wild-type Escherichia coli strain *E*. *coli* K12 (W3110) and a high-yield L-threonine production strain *E. coli* CGMCC 7.232 (preserved in China General Microbiological Culture Collection Center). The constructed plasmid pKOV*-yebN*^{(G74A)} was transferred into *E. coli* K12 (W3110) and *E. coli* CGMCC 7.232, respectively, and through allele replacement, the base G mutated to the base A at the 74^{th} site of the *yebN* gene sequences in the chromosomes of the two strains.

The specific process was as follows: transforming the plasmid pKOV*-yebN*^{(G74A)} into host bacterium competent cells through an electrotransformation process, and adding the cells into 0.5 mL of a SOC liquid culture medium; resuscitating the mixture in a shaker at 30 °C and 100 rpm for 2 h; coating an LB solid culture medium having a chloramphenicol content of 34 µg/mL with 100 µL of the culture solution, and culturing at 30 °C for 18 h; selecting grown monoclonal colonies, inoculating the colonies in 10 mL of an LB liquid culture medium, and culturing at 37 °C and at 200 rpm for 8 h; coating an LB solid culture medium having a chloramphenicol content of 34 µg/mL with 100 µL of the culture solution, and culturing at 42 °C for 12 h; selecting 1-5 single colonies, inoculating the colonies in 1 mL of an LB liquid medium, and culturing at 37 °C and 200 rpm for 4 h; coating an LB solid culture medium containing 10% of sucrose with 100 uL of the culture solution, and culturing at 30 °C for 24 h; selecting monoclonal colonies, and streaking the colonies on an LB solid culture medium having a chloramphenicol content of 34 µg/mL and an LB solid culture medium in a one-to-one correspondence manner; and selecting strains which grew on the LB solid culture medium and could not grow on the LB solid culture medium having the chloramphenicol content of 34 µg/mL for PCR amplification identification. The primers (synthesized by Shanghai Invitrogen Corporation) for use in PCR amplification were as follows:
P5: 5' CCATCACGGCTTGTTGTTC 3' (SEQ ID NO: 31); and
P6: 5' ACGAAAACCCTCAATAATC 3' (SEQ ID NO: 32).

PCR system: 10 × Ex Taq buffer 5 µL, dNTP mixture (2.5 mM each) 4 µL, Mg²⁺ (25 mM) 4 µL, primers (10 pM) 2 µL each, Ex Taq (5 U/µL) 0.25 µL, total volume 50 µL, wherein the PCR amplification was performed as follows: pre-denaturation at 94 °C for 5 min, (denaturing at 94 °C for 30 s, annealing at 52 °C for 30 s, and extending at 72 °C for 30 s, for 30 cycles), and over-extending at 72 °C for 10 min. SSCP (Single-Strand Conformation Polymorphism) electrophoresis was performed on the PCR-amplified product; the amplified fragment of the plasmid pKOV*-yebN*^{(G74A)} was taken as a positive control, the amplified fragment of the wild-type Escherichia coli was taken as a negative control, and water was taken as a blank control. In SSCP electrophoresis, single-stranded oligonucleotide chains having the same length but different sequence arrangements formed different spatial structures in an ice bath and also had different mobilities during electrophoresis. Therefore, the fragment electrophoresis position was not consistent with that of negative control, and a strain having a fragment electrophoresis position consistent with that of positive control is the successfully allele-replaced strain. PCR amplification was performed on the target fragment by taking the successfully allele-replaced strain as a template and using primers P5 and P6, and then the target fragment was ligated to a pMD19-T vector for sequencing. Through sequence comparison of a sequencing result, a recon formed by the base G mutating to the base A at the 74^{th} site in the *yebN* gene coding region sequence is the successfully modified strain. The recon derived from *E. coli* K12 (W3110) was named as YPThr05, and the recon derived from *E. coli* CGMCC 7.232 was named as YPThr 06.

### (3) Threonine Fermentation Experiment

The *E. coli* K12 (W3110) strain, the *E. coli* CGMCC 7.232 strain, and the mutant strains YPThr05 and YPThr06 were inoculated in 25 mL of a liquid culture medium described in Table 1, respectively, and cultured at 37 °C and 200 rpm for 12 h. Then, 1 mL of the resulting culture of each strain was inoculated in 25 mL of a liquid culture medium described in Table 1, and subjected to fermentation culture at 37 °C and 200 rpm for 36 h. The content of L-threonine was determined by HPLC, three replicates of each strain were taken, the average was calculated, and the results are shown in Table 2.

**Table 1 Culture medium formula**

| Component | Formula g/L |
|---|---|
| Glucose | 40 |
| Ammonium sulfate | 12 |
| Potassium dihydrogen phosphate | 0.8 |
| Magnesium sulfate heptahydrate | 0.8 |
| Ferrous sulfate heptahydrate | 0.01 |
| Manganese sulfate monohydrate | 0.01 |
| Yeast extract | 1.5 |
| Calcium carbonate | 0.5 |
| L-methionine | 0.5 |
| pH value adjusted with potassium hydroxide | pH 7.0 |

**Table 2 Threonine fermentation results**

| Strains | Fermentation volume (g/L) | Mean value (g/L) | Multiple of improvement |
|---|---|---|---|
| *E. coli* K12 (W3110) | 0.02 | 0.02 | - |
| | 0.03 | | |
| | 0.02 | | |
| YPThr05 | 2.2 | 2.2 | 110 |
| | 2.1 | | |
| | 2.3 | | |
| *E. coli* CGMCC 7.232 | 16.0 | 16.2 | - |
| | 16.3 | | |
| | 16.2 | | |
| YPThr06 | 17.6 | 17.8 | 9.9% |
| | 17.9 | | |
| | 18.0 | | |

As can be seen from the results of Table 2, the substitution of glycine at the 25^{th} site of the amino acid sequence of the *yebN* gene with aspartic acid contributes to the improvement of the yield of L-threonine for the original strain producing L-threonine with either high or low yield.

## Claims

1. A *spoT* gene, comprising a nucleotide sequence shown in SEQ ID NO: 14.

2. A recombinant protein, comprising an amino acid sequence shown in SEQ ID NO: 16.

3. A recombinant vector, comprising the *spoT gene* according to claim 1.

4. The recombinant vector according to claim 3, wherein the recombinant vector is constructed by introducing the *spoT gene* into a plasmid.

5. A recombinant strain, comprising the *spoT gene* according to claim 1.

6. The recombinant strain according to claim 5, comprising the recombinant vector according to claim 3.

7. A construction method for the recombinant strain according to claim 5, comprising the following steps:
(1) modifying the nucleotide sequence of the wild-type gene shown in SEQ ID NO: 13 to obtain a mutated nucleotide sequence shown in SEQ ID NO: 14;
(2) ligating the mutated nucleotide sequence to a plasmid to construct a recombinant vector, preferably, the plasmid being a pKOV plasmid; and
(3) introducing the recombinant vector into a host strain to obtain the recombinant strain.

8. Use of the *spoT gene* according to claim 1, the recombinant protein according to claim 2, the recombinant vector according to claim 3 or the recombinant strain according to claim 5 in the fermentation preparation of L-threonine.

## Patentansprüche

1. *spoT-Gen,* das eine in SEQ ID NO: 14 gezeigte Nukleotidsequenz umfasst.

2. Rekombinantes Protein, umfassend eine in SEQ ID NO: 16 gezeigte Aminosäuresequenz.

3. Rekombinanter Vektor, umfassend das *spoT-*Gen nach Anspruch 1.

4. Rekombinanter Vektor nach Anspruch 3, wobei der rekombinante Vektor durch Einführen des *spoT*-Gens in ein Plasmid konstruiert wird.

5. Rekombinanter Stamm, umfassend das *spoT*-Gen nach Anspruch 1.

6. Rekombinanter Stamm nach Anspruch 5, umfassend den rekombinanten Vektor nach Anspruch 3.

7. Konstruktionsverfahren für den rekombinanten Stamm nach Anspruch 5, das die folgenden Schritte umfasst:
(1) Modifizieren der Nukleotidsequenz des in SEQ ID NO: 13 gezeigten Wildtyp-Gens, um eine mutierte Nukleotidsequenz zu erhalten, die in SEQ ID NO: 14 gezeigt ist;
(2) Ligieren der mutierten Nukleotidsequenz an ein Plasmid, um einen rekombinanten Vektor zu konstruieren, wobei das Plasmid vorzugsweise ein pKOV-Plasmid ist; und
(3) Einführen des rekombinanten Vektors in einen Wirtsstamm, um den rekombinanten Stamm zu erhalten.

8. Verwendung des *spoT*-Gens nach Anspruch 1, des rekombinanten Proteins nach Anspruch 2, des rekombinanten Vektors nach Anspruch 3 oder des rekombinanten Stamms nach Anspruch 5 bei der Fermentationsherstellung von L-Threonin.

## Revendications

1. Gène *spoT,* comprenant une séquence nucléotidique représentée dans SEQ ID NO : 14.

2. Protéine recombinante, comprenant une séquence d'acides aminés représentée dans SEQ ID NO : 16.

3. Vecteur recombinant, comprenant le gène *spoT* selon la revendication 1.

4. Vecteur recombinant selon la revendication 3, dans lequel le vecteur recombinant est construit en introduisant le gène *spoT* dans un plasmide.

5. Souche recombinante, comprenant le gène *spoT* selon la revendication 1.

6. Souche recombinante selon la revendication 5, comprenant le vecteur recombinant selon la revendication 3.

7. Procédé de construction de la souche recombinante selon la revendication 5, comprenant les étapes suivantes :
(1) modifier la séquence nucléotidique du gène de type sauvage représentée dans SEQ ID NO : 13 pour obtenir une séquence nucléotidique mutée représentée dans SEQ ID NO : 14 ;
(2) ligaturer la séquence nucléotidique mutée à un plasmide pour construire un vecteur recombinant, de préférence, le plasmide étant un plasmide pKOV ; et
(3) introduire le vecteur recombinant dans une souche hôte pour obtenir la souche recombinante.

8. Utilisation du gène *spoT* selon la revendication 1, de la protéine recombinante selon la revendication 2, du vecteur recombinant selon la revendication 3 ou de la souche recombinante selon la revendication 5 dans la préparation par fermentation de L-thréonine.
